# EUROPÄISCHE PATENTSCHRIFT

(11) **EP 3 585 440 B1**
(45) Veröffentlichungstag und Bekanntmachung des Hinweises auf die Patenterteilung: **01.09.2021**
(21) Anmeldenummer: 18714674.1
(22) Anmeldetag: 26.02.2018
(51) Int. Cl.: A61K 47/32, A61K 9/70, A61K 31/465

(54) **NICOTIN ENTHALTENDES TRANSPARENTES TRANSDERMALES THERAPEUTISCHES SYSTEM**
NICOTINE-CONTAINING TRANSPARENT TRANSDERMAL THERAPEUTIC SYSTEM
SYSTÈME THÉRAPEUTIQUE TRANSDERMIQUE TRANSPARENT CONTENANT DE LA NICOTINE

(30) Priorität: 27.02.2017 DE 102017104026
(43) Veröffentlichungstag der Anmeldung: 01.01.2020
(73) Patentinhaber: LTS Lohmann Therapie-Systeme AG, 56626 Andernach (DE)
(72) Erfinder: HILLE, Thomas, 56567 Neuwied (DE); WAUER, Gabriel, 53474 Ahrweiler (DE); BOTZEM, Petra, 56626 Andernach (DE); SEIBERTZ, Frank, 53498 Bad Breisig (DE)
(74) Vertreter: Held, Stephan
(86) Internationale Anmeldenummer: PCT/DE2018/100168
(87) Internationale Veröffentlichungsnummer: WO 2018/153413

(56) Entgegenhaltungen:
- US-A- 4 908 213

## Beschreibung

Die Erfindung betrifft ein den Wirkstoff Nicotin enthaltendes transdermales therapeutisches System (TTS), ein Verfahren zur Herstellung des TTS sowie die Verwendung eines speziellen Polymers zur Stabilisierung von Nicotin in dem TTS.

TTS mit Nicotin sind im Stand der Technik bekannt. Diese sind unter den Handelsnamen Nicotinell (Habitrol), Nicorette oder Niquitin bereits seit Jahren als Arzneimittel zur Bekämpfung der Nicotinabhängigkeit zugelassen und weltweit vermarktet, sind aber nicht in jeder Hinsicht optimal, weil entweder zu große Mengen der giftigen Nicotinbase im getragenen TTS verbleiben oder die Verfärbung der Nicotinbase zu unästhetischen TTS führt. Hierbei ist zu beachten, dass die Lichtempfindlichkeit des Nicotins allgemein bekannt ist und beispielsweise im Merck Index beschrieben wird (Merck Index, 13. Auflage, 6551. Nicotine).

Die Applikation von Arzneimitteln soll dem Bedürfnis des Patienten nach Diskretion Rechnung tragen. Daher sollen insbesondere plakativ deutlich sichtbare TTS vermieden werden. Wegen der Notwendigkeit, TTS international zu vermarkten, wäre es sehr wünschenswert, nicht sogenannte "hautfarbene" lichtundurchlässige Folien als Rückschichten, sondern transparente, also lichtdurchlässige Folien verwenden zu können, um den unterschiedlichen Hautfarben der Menschen in einer globalen Welt Rechnung zu tragen. Die Nachteile der Nicotin TTS gemäß Stand der Technik sollen kurz beschrieben werden:
Das Handelsprodukt Niquitin, ein TTS mit transparenter Rückschicht, das in EP-A-0525105 beschrieben ist, enthält 114 mg Nicotin in einer Matrix aus Polyisobutylen und gibt laut Patienteninformation in 24 Stunden nur 21 mg Wirkstoff ab. Dies sind lediglich 18,4 % des deklarierten Gehalts, was bedeutet, dass 93 mg Nicotin im getragenen TTS verbleiben und in den Hausmüll gelangen. Dies ist wegen der extrem hohen Giftigkeit des Nicotins nicht akzeptabel.

Bei den Handelsprodukten Nicorette und Nicotinell ist die Wirkstoffausnutzung deutlich besser als bei Niquitin. So enthält das 21 mg Nicotin abgebende Nicotinell nur 52,5 mg Nicotin, so dass nur 31,5 mg Nicotin ungenutzt im TTS verbleiben und immerhin 40 % Wirkstoff therapeutisch genutzt werden.

Ähnlich liegen die Verhältnisse bei Nicorette. Dieses TTS wird nur 16 statt 24 Stunden getragen, enthält 40 mg Nicotin und gibt 14 mg ab, was 35 % entspricht und bedeutet, dass nur 26 mg Nicotin ungenutzt im TTS verbleiben. Beide Handelsprodukte haben keine transparente Rückschicht, sondern entweder eine beige lackierte oder eine matte Rückschicht.

Mangelnde Wirkstoffausnutzung wird jedoch nicht nur durch den Wirkstoff verursacht, der im TTS verbleibt, sondern auch bei der Herstellung von TTS, wenn diese aus bahnförmigem Material gestanzt werden, wie dies bei Niquitin und Nicorette der Fall ist, denn pflasterförmige Arzneimittel und Heftpflaster mit abgerundeten Ecken werden aus bahnförmigen Material gestanzt, wobei das gitterförmige, wirkstoffhaltige Material zwischen den Einzelpflastern verworfen und als Sondermüll entsorgt werden muss.

Die US 4,908,213 offenbart ein TTS zur Verabreichung von Nicotin, wobei zusätzlich ein juckreizhemmender Wirkstoff enthalten ist. Als wirkstoffundurchlässige Deckschicht wird vorzugsweise eine metallisierte Polypropylenfolie verwendet.

Die JP 2007 - 262 007 A offenbart ein TTS zur Verabreichung von Nicotin, wobei sichergestellt werden soll, das der Wirkstoff kontinuierlich und konstant abgegeben werden soll.

Die DE 602 01 134 T2 betrifft ein nicotinhaltiges Gel zur Raucherentwöhnung, das für eine langsame und verzögerte Freisetzung von Nikotin sorgen soll.

US 2015/0 190 349 A1 offenbart ein vielschichtiges TTS zur Verabreichung von Nicotin, das gewährleisten soll, dass das TTS stabil auf der Haut des Patienten haftet.

Aufgabe der vorliegenden Erfindung ist daher insbesondere die Bereitstellung eines Nicotin enthaltenden TTS, bei dem eine Verfärbung während der Lagerung vermieden oder zumindest signifikant verringert wird. Auf diese Weise sollen transparente TTS ermöglicht werden, die nicht durch eine solche Verfärbung unansehnlich werden. Dabei soll das Verfahren zu dessen Herstellung beibehalten werden, weil die Wirkstoffausnutzung optimal ist und produktionsbedingte Wirkstoffverluste weitgehend oder vollständig vermieden werden können.

Diese Aufgabe wird überraschenderweise durch ein transdermales therapeutisches System (TTS) mit Nicotin gelöst, das eine für Nicotin undurchlässige Rückschicht, eine wirkstoffhaltige Schicht, umfassend Nicotin als Wirkstoff, wobei das Nicotin als freie Base vorliegt, und mindestens ein Polymer mit mindestens einer Säureamidgruppe als seitenständiger funktioneller Gruppe, wobei das Polymer mit mindestens einer Säureamidgruppe als seitenständiger funktioneller Gruppe ein Vinylpyrrolidon-Vinylacetat-Copolymer ist, und eine ablösbare Schutzschicht.

Die Erfindung betrifft somit ein den Wirkstoff Nicotin enthaltendes transdermales therapeutisches System (TTS) wie in Anspruch 1 definiert. Das erfindungsgemäße TTS zeigt überraschenderweise eine deutlich verlangsamte Verfärbung mit der Zeit gegenüber Nicotin enthaltenden TTS nach dem Stand der Technik, so dass ein Nicotin enthaltendes TTS bereitgestellt werden kann, das sich während der Mindesthaltbarkeit des Arzneimittels von 36 Monaten nicht verfärbt.

Die Erfindung betrifft auch Verfahren zur Herstellung dieser transdermalen therapeutischen Systeme, bei denen in einer bevorzugten Ausführungsform die Systeme mittels eines Druckverfahrens mit Wirkstoff beladen werden, wobei durch ein Verstellungsverfahren, bei dem Einzeldosierung erfolgt, werden Produktionsverluste von wirkstoffhaltigem, gitterförmigem Material vermieden werden, die üblicherweise beim Ausstanzen von Einzelpflastern aus bahnförmigem Material unumgänglich sind. Das gitterförmige Material bezieht sich dabei auf das bei der Vereinzelung durch Schneiden und/oder Stanzen verbleibende überstehende Restmaterial, das in der Regel die geometrische Form eines Gitters aufweist.

Im folgenden wird die Erfindung ausführlich erläutert.

TTS wird als Abkürzung für "transdermales therapeutisches System" verwendet.

Unter einer lichtdurchlässigen Schicht wird eine transparente (= durchsichtige) oder transluzente (= durchscheinende) Schicht verstanden. Eine transparente Schicht lässt Licht fast ungehindert passieren, wogegen eine transluzente Schicht den größten Teil des Lichtes passieren lässt, dabei das Licht aber diffus streut.

Eine Oberfläche wird als matt bezeichnet, wenn sie nur wenige Glanzeinheiten (GE) auf der Glanzgradskala erreicht, die von 100 GE für den Glanz des Schwarzglasstandards bis zu 0 GE für eine absolut matte Oberfläche reicht. Hierfür kann der Reflektometerwert der Oberfläche durch eine Glanzmessung mit einem Reflektometer in 85°C Geometrie bestimmt werden. Eine matte Oberfläche weist bevorzugt einen Reflektometerwert < 10 Glanzeinheiten (GE) auf.

Die Schichten einer bevorzugten Ausführungsform des erfindungsgemäßen TTS sind klar durchsichtig. In einer bevorzugten Ausführungsform ist das erfindungsgemäße TTS transparent, insbesondere transparent und farblos. In einer alternativen Ausführungsform kann das TTS auch eine Rückschicht mit matter Oberfläche aufweisen.

Die Transparenz eines Materials wie eines TTS oder einer Folie bzw. Schicht lässt sich durch Ermittlung des durch das Material transmittierten bzw. absorbierten Lichts bestimmen, etwa mittels eines Macbeth 1500/Plus color-Messsystems (Kollmorgen Instruments Corp., Newburgh, N.Y., USA). Der Prozentsatz des einfallenden Lichts, der bei Durchgang durch das Material absorbiert wird, ist der Opazitätsindex.

Ein Material wie ein TTS oder eine Folie bzw. Schicht wird hier als transparent angesehen, wenn der Opazitätsindex kleiner als 50% ist. Das TTS oder das TTS, von dem die Ablösfolie entfernt worden ist, weist in einer bevorzugten Ausführungsform einen Opazitätsindex kleiner 50% und bevorzugt kleiner 35% auf. Die Rückschicht weist in einer bevorzugten Ausführungsform einen Opazitätsindex von kleiner 50%, bevorzugter kleiner 35% und besonders bevorzugt von kleiner 20% auf.

Sofern nicht anders angeführt, sind für alle nachstehend genannten Polymere Polymere bevorzugt, die pharmazeutisch akzeptabel sind.

Transdermale therapeutische Systeme sind Systeme zur kontrollierten Verabreichung von pharmazeutischen Wirkstoffen über die Haut. Sie werden seit längerem zur Behandlung unterschiedlicher Krankheiten, körperlicher wie geistiger Funktionsstörungen, Beschwerden sowie Unpässlichkeiten eingesetzt. Bei transdermalen therapeutischen Systemen handelt es sich in der Regel um schichtförmig aufgebaute Erzeugnisse in Form von Pflastern, die eine wirkstoffundurchlässige Rückschicht, mindestens eine wirkstoffhaltige Reservoiroder Matrixschicht, gegebenenfalls eine die Geschwindigkeit der Wirkstofffreisetzung kontrollierende Membran und eine ablösbare Schutzschicht, die vor Gebrauch des TTS von diesem abgezogen wird, umfassen.

Die Erfindung betrifft ein transdermales therapeutisches System (TTS), umfassend
a) eine für Nicotin undurchlässige Rückschicht,
b) eine wirkstoffhaltige Schicht, umfassend Nicotin als Wirkstoff, wobei das Nicotin als freie Base vorliegt, und mindestens ein Polymer mit mindestens einer Säureamidgruppe als seitenständiger funktioneller Gruppe, wobei das Polymer mit mindestens einer Säureamidgruppe als seitenständiger funktioneller Gruppe ein Vinylpyrrolidon-Vinylacetat-Copolymer ist, und
c) eine ablösbare Schutzschicht.

Die wirkstoffhaltige Schicht umfasst Nicotin als Wirkstoff. Das Nicotin liegt als freie Base vor. Es liegen also keine protonierten Formen bzw. Salzformen vor. Reines Nicotin ist bei Zimmertemperatur eine farblose, ölige Flüssigkeit, die sich an der Luft rasch braun färbt.

Das TTS enthält z.B. 10 bis 400 mg, vorzugsweise 15 bis 300 mg, insbesondere 20 bis 150 mg Nicotin.

Die wirkstoffhaltige Schicht umfasst ferner mindestens ein Polymer mit mindestens einer Säureamidgruppe als seitenständiger funktioneller Gruppe. Das Polymer ist ein organisches Polymer. Das Polymer ist ein pharmazeutisch akzeptables Polymer mit mindestens einer Säureamidgruppe als seitenständiger funktioneller Gruppe.

Die Säureamidgruppe liegt als seitenständige funktionelle Gruppe vor, d.h. die Säureamidgruppe befindet sich in einer Seitenkette des Polymers. Im Unterschied dazu weisen z.B. Polyamide eine Säureamidgruppe in der Hauptkette auf. Die Säureamid-Gruppe, die auch als Amidgruppe bezeichnet wird, weist allgemein die Struktureinheit -NR-C(=O)- auf, wobei R Wasserstoff oder eine organische Gruppe wie z.B. substituiertes oder unsubstituiertes Alkyl oder substituiertes oder unsubstituiertes Aryl sein kann. Bei der Säureamidgruppe handelt es sich um eine Lactamgruppe, d.h. eine cyclische Säureamidgruppe.

Das Stickstoffatom der Säureamidgruppe, insbesondere das Stickstoffatom der Lactamgruppe, ist direkt an ein Kohlenstoffatom der Hauptkette, des Polymers gebunden. Das Polymer mit mindestens einer Säureamidgruppe als seitenständiger funktioneller Gruppe ist ein N-Vinyllactam-Copolymer.

Ferner offenbart sind Polymere, wobeidie Säureamidgruppe über den Carbonyl-Kohlenstoff der Säureamidgruppe direkt an die Hauptkette, insbesondere ein Kohlenstoffatom der Hauptkette, des Polymers gebunden ist. Beispiele sind ein Acrylamid-Homopolymer oder ein Acrylamid-Copolymer. Es ist auch möglich, dass die Säureamidgruppe nicht direkt an die Hauptkette des Polymers, sondern sich in der Seitenkette befindet und über eine Verknüpfungsgruppe wie z.B. eine Alkylengruppe an die Hauptkette gebunden ist.

Das Polymer mit mindestens einer Säureamid-Gruppe als seitenständiger funktioneller Gruppe ist ein Copolymer, d.h. aus einem oder mehreren Monomeren gebildet sein. Es weist nur ein Teil des Wiederholungseinheiten bzw.

Monomereinheiten des Polymers eine Säureamidgruppe als seitenständige funktionelle Gruppe auf.

Der Anteil an Monomeren, die eine Säureamidgruppe als seitenständige funktionelle Gruppe aufweisen, bezogen auf alle Monomere, die das Polymer bilden, liegt bevorzugt im Bereich von 30 bis 100 Gew.-%, bevorzugt 50 bis 100 Gew.-%. Der Anteil an Monomeren, die keine Säureamidgruppe als seitenständige funktionelle Gruppe aufweisen, liegt dementsprechend im Bereich von 0 bis 70 Gew.-%, bevorzugt 0 bis 50 Gew.-%. Bei einem Copolymer, das auch einen Teil an Monomeren enthält, die keine Säureamidgruppe als seitenständige Gruppe aufweisen, liegt das Gewichtsverhältnis von einem oder mehreren Monomeren, die eine Säureamidgruppe als seitenständige funktionelle Gruppe aufweisen, zu einem einem oder mehreren Monomeren, die keine Säureamidgruppe als seitenständige funktionelle Gruppe aufweisen, bevorzugt im Bereich von 80:20 bis 30:70, bevorzugter 70:30 bis 50:50. Dies gilt insbesondere für ein Vinylpyrrolidon-Vinylacetat-Copolymer.

Das Polymer kann aus einem oder mehreren Monomeren, die eine SäureamidGruppe als seitenständige funktionelle Gruppe aufweisen, und gegebenenfalls einem oder mehreren Monomeren, die keine Säureamid-Gruppe als seitenständige funktionelle Gruppe aufweisen, gebildet werden.

Beispiele für Monomere, die eine Säureamid-Gruppe als seitenständige funktionelle Gruppe aufweisen, sind Acrylamid und N-Vinylamide, insbesondere N-Vinyllactame. Beispiele für N-Vinylamide bzw. N-Vinyllactame sind N-Vinylamid, N-Vinylmethylacetamid, Vinylethylacetamid, N-Vinylmethyl-isobutyramid, N-Vinyl-2-pyrrolidon, N-Vinyl-3-pyrrolidon, N-Vinyl-2-piperidon, N-Vinyl-caprolactam, N-Vinyl-5-methyl-2-pyrrolidon, N-Vinyl-3-methyl-2-pyrrolidon und N-Vinylimide, wie N-Vinylsuccinimid und N-Vinylphthalimid. N-Vinyl-2-pyrrolidon ist besonders bevorzugt.

Beispiele für Monomere, die keine Säureamid-Gruppe als seitenständige funktionelle Gruppe aufweisen, sind Vinylacetat oder N-Vinylimidazol.

Beispiele für Homopolymere, die eine Säureamid-Gruppe als seitenständige funktionelle Gruppe aufweisen, sind Polyacrylamid und Poly-N-vinylamide, insbesondere Poly-N-vinyllactame. Beispiele für Poly-N-vinylamide bzw. Poly-N-vinyllactame sind Poly-N-vinylamid, Poly-N-vinylmethylacetamid, Poly-N-vinylethylacetamid, Poly-N-vinylmethyl-isobutyramid, Poly-N-vinyl-2-pyrrolidon, Poly-N-vinyl-3-pyrrolidon, Poly-N-vinyl-2-piperidon, Poly-N-vinyl-caprolactam, Poly-N-vinyl-5-methyl-2-pyrrolidon, Poly-N-vinyl-3-methyl-2-pyrrolidon und Poly-N-vinylimide, wie Poly-N-vinylsuccinimid und Poly-N-vinylphthalimid.

Das Polymer mit mindestens einer Säureamidgruppe als seitenständiger funktioneller Gruppe ist ein Vinylpyrrolidon-Copolymer, wobei das Copolymer aus Vinylpyrrolidon und dem Co-Monomer Vinylacetat gebildet ist. Sofern nicht anders angegeben, handelt es sich bei Vinylpyrrolidon bevorzugt um N-Vinyl-2-pyrrolidon, wie in der Technik üblich.

Das Polymer ist ein Vinylpyrrolidon-Vinylacetat-Copolymer, das partiell hydrolysiert sein kann, insbesondere ein N-Vinyl-2-pyrrolidon-Vinylacetat-Copolymer, das partiell hydrolysiert sein kann. Die partielle Hydrolyse bezieht sich dabei auf die teilweise Hydrolyse der Acetatgruppe. Polyvinylpyrrolidon wird auch als PVP oder Povidin bezeichnet und ist im Handel z.B. unter dem Handelsnamen Kollidon von BASF erhältlich. Das Polymer mit mindestens einer Säureamidgruppe als seitenständiger funktioneller Gruppe ist bevorzugt wasserlöslich. Ein geeignetes Vinylpyrrolidon-Vinylacetat-Copolymer wird z.B. von BASF unter dem Handelsnamen Kollidon VA 64 (Gewichtsverhältnis von Vinylpyrrolidon zu Vinylacetat von 60 zu 40) vertrieben. Vinylpyrrolidon-Vinylacetat-Copolymere sind auch von Ashland Inc., USA, kommerziell erhältlich.

Die mittlere Molmasse von löslichen Polyvinylpyrrolidonen wird in den gängigen Pharmacopeien Ph.Eur., USP und JP über den K-Wert beschrieben. Dieser Wert wird über die relative Viskosität von wässrigen Polyvinylpyrrolidon-Lösungen errechnet und ist bei Polyvinylpyrrolidonen der BASF immer Teil des Handelsnamen. So hat PVP K 90 einen mittleren K-Wert von 90 (81,0 - 97,2).

Grundlage der viskosimetrischen Untersuchungsmethoden ist die Erkenntnis, dass eine Flüssigkeit durch ein eingebrachtes Teilchen eine Viskositätserhöhung erfährt, die proportional dem Volumen des Teilchens ist. Da bei polymerhomologen Reihen das Volumen der Makromoleküle mit der Molmasse zunimmt, muss ein Zusammenhang zwischen der Viskositätserhöhung und Molmasse bestehen. Die relative Viskositätserhöhung bezeichnet man im Allgemeinen als spezifische Viskosität. Der k-Wert ist somit die spezifische Viskosität von Polyvinylpyrrolidon-Lösungen, wobei der Feststoffanteil 1 bzw. 5 % beträgt.

Die Menge an dem mindestens einen Polymer mit mindestens einer Säureamidgruppe als seitenständiger funktioneller Gruppe in dem TTS beträgt z.B. mindestens 10 mg, bevorzugt mindestens 15 mg, und/oder z.B. nicht mehr als 100 mg, bevorzugt nicht mehr als 60 mg.

Das Gewichtsverhältnis von Nikotin zu dem mindestens einen Polymer mit mindestens einer Säureamidgruppe als seitenständiger funktioneller Gruppe in dem TTS beträgt vorzugsweise 10 : 1 bis 1 : 2, besonders bevorzugt 5 : 1 bis 1 : 1, noch mehr bevorzugt 3 : 1 bis 1 : 1.

Überraschenderweise gelingt es durch die Zugabe des Polymers mit mindestens einer Säureamidgruppe als seitenständiger funktioneller Gruppe das im TTS enthaltene Nicotin so zu stabilisieren, dass es sich nicht oder deutlich langsamer verfärbt. Neben dieser Stabilisierungsfunktion dient das Polymer auch zur Einstellung einer geeigneten Viskosität der wirkstoffhaltigen Zusammensetzung, die bei der bevorzugten Herstellung des TTS für die Einzeldosierung nötig ist.

Die vorstehend genannte Handelsprodukte Kollidon (Polyvinylpyrrolidon) oder Kollidon VA 64 (Vinylpyrrolidon-Vinylacetat-Copolymer) sind als pharmazeutische Hilfsstoffe in den gängigen Arzneibüchern Ph.Eur. USP und JPE monographiert; siehe auch z.B. Bühler, Volker, Kollidon® Polyvinylpyrrolidone excipients for the pharmaceutical industry BASF SE 9. Auflage, März 2008. Beide Polymertypen bilden mit protonischen Stoffen bzw. Säuren über Wasserstoffbrückenbindungen Additionsverbindungen, ihr Einsatz zur Stabilisierung von aprotisch-polaren oxidationsempfindlichen Stoffen war jedoch bisher nicht bekannt, so dass ihr Effekt zur Stabilisierung des Nicotins umso überraschender ist und eine Innovation auf dem Arzneimittelsektor darstellt.

Erstaunlicherweise bewirkt der Einsatz der Polymere mit mindestens einer Säureamidgruppe als seitenständiger funktioneller Gruppe eine Vermeidung oder Verlangsamung der Verfärbung des Nicotins mit der Zeit, so dass es möglich wird, eine lichtdurchlässige Schicht bzw. Folie als Rückschicht zu verwenden, ohne dass unansehnliche Verfärbungen sichtbar werden.

Das TTS umfasst eine für Nicotin undurchlässige Rückschicht. Die Rückschicht eines TTS muss für den im TTS enthaltenen Wirkstoff undurchlässig sein, um ein unerwünschtes Austreten des Wirkstoffs aus der Seite des TTS, die der Haut abgewandt ist, zu verhindern.

Die Rückschicht des TTS ist vorzugsweise lichtdurchlässig, insbesondere transparent. In einer alternativen Ausführungsform kann die Rückschicht matt sein, so dass im TTS die nach außen weisende Oberfläche der Rückschicht eine matte Oberfläche ist.

Am zweckmäßigsten sind sind für die Rückschicht Schichten oder Folien aus Kunststoff, wie z.B. Polyethylenterephthalat (PET). Der Vorteil dieser Kunststoffschichten oder Kunststofffolien liegt darin, dass sie kostengünstig herzustellen und gegenüber nahezu allen pharmazeutischen Wirkstoffen undurchlässig sind. Bevorzugt ist die für Nicotin undurchlässige Rückschicht eine Kunststofffolie, insbesondere eine transparente Kunststofffolie.

Als Kunststoffe für die wirkstoffundurchlässige Rückschicht, insbesondere für die wirkstoffundurchlässige transparente Rückschicht, eignen sich Polyester, vor allem Polyester, die sich durch besondere Festigkeit auszeichnen, wie z. B. Polyethylenterephthalat und Polybutylenterephthalat, darüber hinaus aber auch andere hautverträgliche Kunststoffe wie Acrylnitril-Methylacrylat-Copolymere, wie z.B. Barex®-Folien von arbo plastic AG, Schweiz. Für die Rückschicht können auch Verbundlaminate aus zwei oder mehr Kunststoffolien verwendet werden.

Für die Rückschicht wird besonders bevorzugt eine Folie aus Polyethylenterephthalat (PET), insbesondere eine transparente Folie aus PET, verwendet. Eine große Anzahl an unterschiedlichen Typen von geeigneten PET-Folien sind von Mitsubishi Polyester Film GmbH unter dem Markennamen Hostaphan® im Handel erhältlich.

Als Rückschicht werden im Stand der Technik häufig Schichten umfassend metallisches Aluminium verwendet, insbesondere in Form von Verbundlaminaten von Aluminiumfolie und Kunststoffolien. Dies ist aber erfindungsgemäß nicht bevorzugt, d.h. in der Rückschicht ist bevorzugt kein metallisches Aluminium, z.B. in Form einer Aluminiumfolie, enthalten. Die Rückschicht ist ferner bevorzugt frei oder im wesentlichen frei von Farbpigmenten und Farbstoffen.

Die Rückschicht ist bevorzugt farblos, insbesondere transparent und farblos. Die Rückschicht ist besonders bevorzugt klar bzw. klarsichtig. Geeignete transparente Folie bzw. Klarsichtfolien sind im Handel erhältlich.

Das TTS umfasst ferner eine ablösbare Schutzschicht. Solche ablösbaren Schutzschichten sind im Handel erhältlich. Die ablösbare Schutzschicht ist ebenfalls für Nicotin undurchlässig.

Für die ablösbare Schutzschicht können grundsätzlich dieselben Materialien verwendet werden wie für die Rückschicht, vorausgesetzt, dass sie durch eine geeignete Oberflächenbehandlung, wie z. B. Silikonisierung, ablösbar ausgerüstet ist. Es können aber auch andere ablösbaren Schutzschichten wie z. B. mit Polytetrafluorethylen behandeltes Papier oder ®Cellophan (Cellulosehydrat) verwendet werden.

Die im Folgenden diskutierten Schichten und Fixiereinrichtungen sind im TTS zwischen der für Nicotin undurchlässigen Rückschicht und der ablösbaren Schutzschicht angeordnet.

Zur Befestigung eines transdermalen therapeutischen Systems auf der Haut sowie zur Gewährleistung der kontrollierten Verabreichung des Wirkstoffs ist das TTS insbesondere mit einer haftklebenden Schicht versehen. Diese haftklebende Schicht kann z.B. mit der mit der nachstehend beschriebenem Matrixschicht oder der hautseitigen wirkstoffhaltigen Schicht identisch sein, kann aber auch zusätzlich vorhanden sein, sofern die wirkstoffhaltige Schicht oder eine optional vorhandene Membran oder die Matrixschicht nicht haftklebend ist.

Somit umfasst das erfindungsgemäße transdermale therapeutische System insbesondere eine Schicht oder Fixiereinrichtung, die haftklebend ist und auf der Oberfläche der ablösbaren Schutzschicht, die der für Nicotin undurchlässigen Rückschicht zugewandt ist, angeordnet ist, wobei es sich bei der haftklebenden Schicht oder Fixiereinrichtung z.B. um die wirkstoffhaltige Schicht oder eine davon verschiedene haftklebende Schicht oder Fixiereinrichtung handeln kann. Diese von der wirkstoffhaltigen Schicht verschiedene haftklebende Schicht oder Fixiereinrichtung kann z.B. die nachstehend erläuterte Matrixschicht oder eine zusätzliche haftklebende Schicht oder Fixiereinrichtung sein. Welche Schicht oder Fixiereinrichtung auf der Oberfläche der ablösbaren Schutzschicht, die der Rückschicht zugewandt ist, angeordnet und haftklebend ist, hängt vom jeweiligen Typ des TTS ab wie später erläutert. Es können gegebenenfalls auch andere Schichten haftklebend sein.

Die ablösbare Schutzschicht wird bei der Anwendung abgelöst, und das von der ablösbaren Schutzschicht befreite TTS wird dann mit der vorstehend angeführten Schicht oder Fixiereinrichtung, die haftklebend ist, auf die gewünschte Stelle auf der Haut aufgeklebt.

Unabhängig davon, ob es sich bei der Schicht oder Fixiereinrichtung, die haftklebend ausgebildet ist, um die wirkstoffhaltige Schicht, die Matrixschicht oder eine zusätzliche haftklebende Schicht oder Fixiereinrichtung handelt, umfasst die Schicht oder Fixiereinrichtung, die haftklebend ist, insbesondere einen Haftklebstoff. Der Haftklebstoff basiert auf mindestens einem Polymer. Solche Polymere sind in der Technik gut bekannt. Geeignete Beispiele für Polymere für den Haftklebstoff sind im Folgenden angeführt.

Der Haftklebstoff umfasst vorzugsweise mindestens ein Polymer ausgewählt aus Poly(meth)acrylaten, Polyisobutylen, Polyvinylacetat, Ethylen-Vinylacetat-Copolymer, natürlichen und/oder synthetischen Kautschuken, Styrol-Dien-Copolymeren wie Styrol-Butadien-Blockcopolymeren, Polyestern, Polychloroprenen, Polyvinylethern, Polyurethanen, Silikonpolymeren, die auch als Polysiloxane bezeichnet werden, oder einen Heißschmelzkleber.

Beispiele für natürliche und/oder synthetische Kautschuke sind Acrylnitril-Butadien-Kautschuk, Butylkautschuk oder Neoprenkautschuk. Poly(meth)acrylate sind Polymere von einem oder mehreren Monomeren ausgewählt aus Acrylsäureestern und/oder Methacrylsäureestern und/oder Acrylsäure und/oder Methacrylsäure und gegebenfalls zusätzlichen Comonomeren wie z.B. Vinylacetat, wobei bevorzugt mindestens ein Acrylsäureester oder Methacrylsäureester enthalten ist. Bevorzugt sind Polyacrylate von einem oder mehreren Acrylsäureestern und gegebenenfalls Acrylsäure und/oder einem zusätzlichen Comonomer wie z.B. Vinylacetat. Das Siliconpolymer kann z.B. ein Silicongummi sein.

Bei den Polymeren für den Haftklebstoff handelt es sich insbesondere um Polymere mit einer Glasübergangstemperatur (Tg) < 0°C, die als matrixbildende Polymere geeignet sind. Das mindestens eine Polymer für den Haftklebstoff ist bevorzugt lichtdurchlässig oder transparent. Der Haftklebstoff kann neben dem vorstehenden aufgeführten Polymer gegebenenfalls ferner weitere Bestandteile, z.B. mindestens ein Harz und/oder Weichmacher, enthalten. Ein Beispiel hierfür sind Triglyceride von Fettsäuren.

Die zusätzliche haftklebende Schicht oder Fixiereinrichtung kann als Schicht ausgebildet sein. Alternativ kann es sich um eine haftklebende Fixiereinrichtung handeln, die zwischen der ablösbaren Schutzschicht und der darüberliegenden Schicht angeordnet ist. Die Fixiereinrichtung kann z.B. durch in der darüberliegenden Schicht eingebettete Haftklebstoffabschnitte, wie z.B. ein umlaufender Kleberand oder auch Klebepunkte, gebildet sein.

Das TTS kann optional zusätzlich eine Membran umfassen. Während die wirkstoffhaltige Schicht oder Matrixschicht insbesondere eine oder mehrere Polymere mit einer Tg < 0°C umfasst, in der Stoffe gelöst werden können, wird eine optionale Membran aus einem oder mehreren Polymeren mit einer Tg > 0°C gebildet. Stoffe werden durch eine Membran aus solchen Polymeren nicht gelöst, weshalb hat die Membran Löcher oder Poren aufweist, durch die der Stoff diffundieren kann. Die Abgaben des Wirkstoffs aus einer Matrixschicht und einer Membran folgen unterschiedlichen Kinetiken. Die Membran, sofern vorhanden, kann z.B. zwischen der wirkstoffhaltigen Schicht oder Matrixschicht und einer Fixiereinrichtung angeordnet sein.

Das erfindungsgemäße TTS kann für die unterschiedlichen Typen von TTS verwendet werden, die in der Technik bekannt sind. Je nach Typ können sich verschiedene Ausführungsformen ergeben, die im Folgenden erläutert werden.

In einer ersten Ausführungsform kann die wirkstoffhaltige Schicht, neben Nicotin und dem mindestens einen Polymer mit mindestens einer Säureamidgruppe als seitenständiger funktioneller Gruppe, zusätzlich mindestens ein Polymer zur Bildung einer Matrix, vorzugsweise einer Klebematrix, umfassen. Die Matrix kann jedoch auch nicht haftklebend sein. Bei dem mindestens einen Polymer zur Bildung einer Matrix kann es sich um ein Polymer für den Haftklebstoff handeln, der vorstehend definiert wurde. Auf die dort angegebenen Beispiele wird verwiesen. Andere geeignete Polymere für das mindestens eine Polymer zur Bildung einer Matrix sind grundsätzlich Polymere mit einer Glasübergangstemperatur Tg < 0°C, weil das Polymer nur dann als Matrix wirkt, wenn es im Gummizustand ist.

Bei der ersten Ausführungsform kann die wirkstoffhaltige Schicht haftklebend sein. Sie umfasst dann vorzugsweise einen Haftklebstoff mit dem mindestens einen Polymer für den Haftklebstoff wie vorstehend definiert. In diesem Fall kann das TTS die Rückschicht, die haftklebende wirkstoffhaltige Schicht und die ablösbare Schutzschicht umfassen bzw. daraus gebildet sein. Eine solches TTS wird auch als monolithisches Matrix-TTS bezeichnet.

Bei der ersten Ausführungsform kann neben der wirkstoffhaltigen Schicht, die das mindestens eine Polymer zur Bildung einer Matrix umfasst, ferner eine zusätzliche haftklebende Schicht oder Fixiereinrichtung umfasst sein, die zwischen der wirkstoffhaltigen Schicht und der ablösbaren Schutzschicht angeordnet ist. Bei dieser Variante braucht die wirkstoffhaltige Schicht nicht notwendigerweise haftklebend zu sein. Die zusätzliche haftklebende Schicht oder Fixiereinrichtung wurde vorstehend definiert und umfasst dann vorzugsweise einen Haftklebstoff mit dem mindestens einen Polymer für den Haftklebstoff wie vorstehend erörtert. Eine solches TTS wird auch als Mehrschicht-Matrix-TTS bezeichnet.

Bei der ersten Ausführungsform beträgt der gemeinsame Anteil an Nicotin und dem mindestens einen Polymer mit mindestens einer Säureamidgruppe als seitenständiger funktioneller Gruppe in der wirkstoffhaltigen Schicht z.B. 1 bis 20 Gew.-%, bevorzugt 5 bis 15 Gew.-%, bezogen auf das Gewicht der wirkstoffhaltigen Schicht.

Bei einer zweiten und bevorzugten Ausführungsform umfasst das TTS zusätzlich eine Matrixschicht zur Steuerung der Abgabe des Wirkstoffs bzw. des Nicotins und gegebenenfalls eine zusätzliche haftklebende Schicht oder Fixiereinrichtung, die zwischen der Matrixschicht und der ablösbaren Schutzschicht angeordnet ist. Die Matrixschicht kann haftklebend sein. In diesem Fall ist die zusätzliche haftklebende Schicht oder Fixiervorrichtung nicht erforderlich. Es ist bei der zweiten Ausführungsform bevorzugt, dass das TTS die haftklebende Schicht oder Fixierungseinrichtung zwischen der ablösbaren Schutzschicht und der Matrixschicht umfasst. In diesem Fall braucht die Matrixschicht nicht notwendigerweise haftklebend zu sein.

Die Matrixschicht und die haftklebende Schicht oder Fixiereinrichtung des erfindungsgemäßen TTS können aus dem gleichen Material oder aus verschiedenen Materialien bestehen.

Die Matrixschicht zur Steuerung der Abgabe des Wirkstoffs umfasst bevorzugt mindestens ein Polymer zur Bildung einer Matrix, vorzugsweise einer Klebematrix. Bei dem mindestens einen Polymer zur Bildung einer Matrix kann es sich um ein Polymer für den Klebstoff handeln, der vorstehend erläutert wurde. Auf die dort angegebenen Beispiele wird verwiesen. Matrixbildende Polymere sind auch haftklebend.

Die Matrixschicht zur Steuerung der Abgabe des Wirkstoffs ist im ursprünglichen Zustand bevorzugt wirkstofffrei.

Die in der zweiten Ausführungsform gegebenenfalls und bevorzugt vorhandene zusätzliche haftklebende Schicht oder Fixiereinrichtung wurde bereits vorstehend definiert und umfasst vorzugsweise einen Haftklebstoff mit dem mindestens einen Polymer für den Haftklebstoff wie vorstehend ausgeführt. Auf die dort angegebenen Beispiele wird verwiesen.

Die Matrixschicht und/oder die zusätzliche haftklebende Schicht oder Fixiereinrichtung, sofern vorhanden, können gegebenenfalls ein Material umfassen, das ausgewählt ist aus kationischen Copolymeren auf Basis von Dimethylaminoethylmethacrylat und neutralen Methacrylsäureestern, und neutralen Copolymeren auf Basis von Butylmethacrylat und Methylmethacrylaten besteht. Ein Beispiel ist Eudragit® E 100 (kationisches Copolymer auf Basis von Dimethylaminoethylmethacrylat, Butylmethacrylat und Methylmethacrylat in einem Verhältnis von 2:1:1).

Wenn die Matrixschicht haftklebend ist, umfasst sie vorzugsweise einen Haftklebstoff mit dem mindestens einen Polymer für den Haftklebstoff wie vorstehend erörtert. Die Matrixschicht kann auch mehrschichtig, z.B. zweischichtig, ausgebildet sein, wobei die Teilschichten z.B. verschiedene Arten von Inhaltsstoffen oder verschiedene Konzentrationen der Inhaltsstoffe enthalten, um z.B. einen Gradienten zu bilden.

Bei der zweiten Ausführungsform kann die wirkstoffhaltige Schicht in der Matrixschicht zur Steuerung der Abgabe des Nicotins eingebettet und/oder auf der der Rückschicht zugewandten Oberfläche der Matrixschicht angeordnet sein. Es ist dabei bevorzugt, dass die wirkstoffhaltige Schicht nur teilflächig in der Rückschicht eingebettet oder auf der Oberfläche der Matrixschicht angeordnet ist, z.B. in einem zentralen Bereich der Matrixschicht. Es ist auch möglich, dass die wirkstoffhaltige Schicht nicht als eine zusammenhängende Schicht, sondern in Form von zwei oder mehr Teilschichten in der Rückschicht eingebettet oder auf der Oberfläche der Matrixschicht angeordnet ist.

Wie oben bereits erwähnt, ist es bevorzugt, dass bei dieser Ausführungsform zwischen der Matrixschicht und der ablösbaren Schutzschicht eine zusätzliche haftklebende Schicht oder Fixiereinrichtung wie vorstehend definiert vorhanden ist.

Bei der zweiten Ausführungsform umfasst die wirkstoffhaltige Schicht neben Nicotin und dem mindestens einen Polymer mit mindestens einer Säureamidgruppe als seitenständiger funktioneller Gruppe bevorzugt nur einen geringen Anteil an weiteren Bestandteilen oder keine weiteren Bestandteile.

Bei der zweiten Ausführungsform beträgt der gemeinsame Anteil an Nicotin und dem mindestens einen Polymer mit mindestens einer Säureamidgruppe als seitenständiger funktioneller Gruppe in der wirkstoffhaltigen Schicht dementsprechend z.B. 1 bis 100 Gew.-%, bevorzugt 5 bis 100 Gew.-%, bevorzugter 5 bis 75 Gew.-%, bezogen auf das Gewicht der wirkstoffhaltigen Schicht. In einer bevorzugten Ausführungsform beträgt der gemeinsame Anteil an Nicotin und dem mindestens einen Polymer mit mindestens einer Säureamidgruppe als seitenständiger funktioneller Gruppe in der wirkstoffhaltigen Schicht 60 bis 100 Gew.-%, bevorzugt 80 bis 100 Gew.-%, bevorzugter 90 bis 100 Gew.-%, bezogen auf das Gewicht der wirkstoffhaltigen Schicht.

Die Matrixschicht steuert die Abgabe des Nicotins. Im ursprünglichen Zustand ist die Matrixschicht vorzugsweise wirkstofffrei. Das in der wirkstoffhaltigen Schicht enthaltene Nicotin diffundiert oder fließt mit der Zeit in die Matrixschicht, bis gegebenenfalls eine Sättigungskonzentration erreicht wird. Bei der Anwendung des TTS verringert sich die Nicotinkonzentration in der Matrixschicht durch Aufnahme durch die Haut. Gegebenenfalls kann dann weiteres Nicotin aus der wirkstoffhaltigen Schicht in die Matrixschicht diffundieren. Die Diffusion von Nicotin in die Matrixschicht beginnt unmittelbar nach der Aufbringung der wirkstoffhaltigen Schicht auf die Matrixschicht. Es versteht sich daher, dass die Zusammensetzung der wirkstoffhaltigen Schicht sich mit der Zeit ändern kann. Die nachstehenden Angaben zur wirkstoffhaltigen Schicht beziehen sich daher insbesondere auf die für die Bildung der wirkstoffhaltigen Schicht verwendete wirkstoffhaltige Zusammensetzung, gelten aber in der Regel auch für die wirkstoffhaltige Schicht nach der Herstellung des TTS.

In der zweiten Ausführungsform handelt es sich bei der wirkstoffhaltigen Schicht vorzugsweise um eine halbfeste Schicht. Die wirkstoffhaltige Schicht ist bevorzugt durch Aufbringen einer wirkstoffhaltigen Zusammensetzung erhältlich, die eine Brookfield-Viskosität im Bereich von 10 bis 100 dPa·s, besonders bevorzugt in einem Bereich von 15 bis 30 dPa·s, aufweist, die bei Raumtemperatur (20 °C) bestimmt wurde.

Das transdermale therapeutische System kann gegebenenfalls mindestens eine Säure, z.B. eine organische Säure, wie Weinsäure und Salicylsäure, oder eine anorganische Säure wie Salzsäure, enthalten, dies ist aber nicht bevorzugt. Das TTS, insbesondere die wirkstoffhaltige Schicht, sind bevorzugt im wesentlichen frei von Säure. Der Anteil an Säure in dem TTS ohne Rückschicht und ablösbare Schutzfolie sollte z.B. nicht mehr als 2 Gew.-%, bevorzugt nicht mehr als 0,5 Gew.-%, bevorzugter nicht mehr als 0,02 Gew.-%, bezogen auf das Gewicht des transdermalen therapeutischen Systems ohne Rückschicht und ablösbarer Schutzfolie, betragen. Dies gilt insbesondere für den Anteil an Säure im TTS ohne Rückschicht und ablösbarer Schutzschicht. Besonders bevorzugt ist das TTS säurefrei, weil Nicotinsalze nicht durch die Haut diffundieren.

Das transdermale therapeutische System kann gegebenenfalls ein oder mehrere Antioxidationsmittel in einer oder mehreren der vorstehend genannten Schichten enthalten. Das TTS ist aber bevorzugt frei von Antioxidationsmitteln, zumindestens in den Schichten, die von der ablösbaren Schutzschicht und der Rückschicht verschieden sind.

Typische Dickenabmessungen für erfindungsgemäße TTS sind: Gesamtdicke von ca. 123 µm bis 5550 µm, vorzugsweise 285 µm bis 1550 µm; Dicke der für Nicotin undurchlässigen Rückschicht von 8 bis 50 µm, vorzugsweise 15 bis 25 µm.

Durch die erfindungsgemäße Stabilisierung des Nicotins durch das Säureamid-haltige Polymer findet keine Verfärbung bzw. nur eine sehr verlangsamte Verfärbung statt. Daher können erfindungsgemäß transparente TTS bereitgestellt werden. In einer besonders bevorzugten Ausführungsform wird daher eine transparente Rückschicht verwendet, wobei auch die weiteren Schichten der TTS lichtdurchlässig sind, abgesehen von der ablösbaren Schutzschicht, die nicht notwendigerweise lichtdurchlässig sein muss. Bei dieser Anwendung ist das TTS gegenüber der Haut, auf der sie haften, fast unsichtbar, da die natürliche Hautfarbe des Anwenders durch das TTS hindurch sichtbar ist.

Die Erfindung betrifft ferner ein Verfahren zur Herstellung eines erfindungsgemäßen transdermalen therapeutischen Systems wie vorstehend beschrieben, wobei das Verfahren folgende Schritte umfasst
- Bereitstellen einer ersten Trägerschicht oder Herstellen eines Ausgangslaminats umfassend eine erste Trägerschicht,
- Aufbringen einer fließfähigen wirkstoffhaltigen Zusammensetzung, umfassend Nicotin als Wirkstoff, wobei das Nicotin als freie Base vorliegt, und mindestens ein Polymer mit mindestens einer Säureamidgruppe als seitenständiger funktioneller Gruppe, wobei das Polymer mit mindestens einer Säureamidgruppe als seitenständiger funktioneller Gruppe ein Vinylpyrrolidon-Vinylacetat-Copolymer ist, auf die erste Trägerschicht oder das Ausgangslaminat und
- Auflaminieren der restlichen Schichten des transdermalen therapeutischen Systems, umfassend eine zweite Trägerschicht, auf die mit der wirkstoffhaltigen Zusammensetzung versehene erste Trägerschicht oder das mit der wirkstoffhaltigen Zusammensetzung versehene Ausgangslaminat,
wobei die transdermalen therapeutischen Systeme durch Schneiden und/oder Stanzen vor oder nach dem Aufbringen der wirkstoffhaltigen Zusammensetzung aus dem bis dahin entstandenen Produkt oder Laminat vereinzelt werden können, und die erste Trägerschicht die ablösbare Schutzschicht, die vorzugsweise für Nicotin undurchlässig ist, und die zweite Trägerschicht die für Nicotin undurchlässige Rückschicht bildet oder umgekehrt.

Der gemeinsame Anteil an Nicotin und dem mindestens einen Polymer mit mindestens einer Säureamidgruppe als seitenständiger funktioneller Gruppe in der fließfähigen wirkstoffhaltigen Zusammensetzung beträgt z.B. 1 bis 100 Gew.-%, bevorzugt 5 bis 100 Gew.-%. In einer weiteren bevorzugten Ausführungsform beträgt der gemeinsame Anteil an Nicotin und dem mindestens einen Polymer mit mindestens einer Säureamidgruppe als seitenständiger funktioneller Gruppe in der fließfähigen wirkstoffhaltigen Zusammensetzung 60 bis 100 Gew.-%, bevorzugt 80 bis 100 Gew.-%, bevorzugter 90 bis 100 Gew.-%, bezogen auf das Gewicht der fließfähigen wirkstoffhaltigen Zusammensetzung.

Die erste Trägerschicht ist bevorzugt die ablösbare Schutzschicht, die vorzugsweise für Nicotin undurchlässig ist. Die zweite Trägerschicht ist bevorzugt die für Nicotin undurchlässige Rückschicht, die bevorzugt transparent ist.

Das Auflaminieren der restlichen Schichten des transdermalen therapeutischen Systems, umfassend eine zweite Trägerschicht, kann dabei durch einzelnes Aufbringen der restlichen Schichten hintereinander oder bevorzugt durch Auflaminieren der restlichen Schichten als Verbund erfolgen. Selbstverständlich kann das Auflaminieren auch durch Kombination von Auflaminieren einer oder mehrerer restlicher Schichten einzeln und/oder von zwei oder mehreren restlichen Schichten im Verbund erfolgen.

Die Vereinzelung durch Schneiden und/oder Stanzen erfolgt bevorzugt nach dem Aufbringen der wirkstoffhaltigen Zusammensetzung, z.B. nachdem alle Schichten des TTS miteinander verbunden wurden. Es ist aber auch möglich, nach dem Aufbringen der wirkstoffhaltigen Zubereitung, jedoch vor dem Aufbringen der Rückschicht, TTS aus dem bis dahin entstandenen Verbundlaminat zu vereinzeln und anschließend mit einer Rückschicht abzudecken.

In einer bevorzugten Ausführungsform umfasst das erfindungsgemäße Verfahren folgende Schritte
- Herstellen des Ausgangslaminats umfassend die erste Trägerschicht und eine Matrixschicht oder einen Teil einer Matrixschicht, wobei gegebenenfalls eine haftklebende Schicht oder Fixiereinrichtung zwischen der ersten Trägerschicht und der Matrixschicht angeordnet wird,
- Aufbringen der fließfähigen wirkstoffhaltigen Zusammensetzung auf die Matrixschicht oder den Teil der Matrixschicht, und
- Auflaminieren der restlichen Schichten des transdermalen therapeutischen Systems, umfassend die zweite Trägerschicht, auf die mit der wirkstoffhaltigen Zusammensetzung versehene Matrixschicht, wobei, sofern das Ausgangslaminat nur einen Teil der Matrixschicht umfasst, der restliche Teil der Matrixschicht als erste Schicht auf die mit der Wirkstoffzusammensetzung versehenen teilweisen Matrixschicht aufgebracht wird.

Der Teil der Matrixschicht bezieht sich dabei auf einen Teil in Dickenrichtung. Es kann z.B. zunächst etwa die Hälfte oder zwei Drittel oder irgendein anderer Teil der Gesamtdicke der Matrixschicht aufgebracht werden und nach Aufbringen der wirkstoffhaltigen Zusammensetzung der restliche Teil der Dicke der Matrixschicht. Das Aufbringen der fließfähigen wirkstoffhaltigen Zusammensetzung erfolgt bevorzugt mittels eines Druckverfahrens einzeln dosierte Portionen der fließfähigen, wirkstoffhaltigen Zusammensetzung auf das Ausgangslaminat oder die Matrixschicht oder den Teil der Matrixschicht aufgebracht werden. Das Aufbringen der einzeln dosierten Portionen erfolgt dabei insbesondere teilflächig.

Bei dem vorstehenden Druckverfahren kann es sich um ein Tampondruckverfahren handeln. Ein solches Verfahren ist z.B. aus US-Patent 5 110 599 bekannt.

Bei dem vorstehenden Druckverfahren kann es sich außerdem um ein Verfahren handeln, bei dem die wirkstoffhaltige Zubereitung durch eine mit mindestens einem Durchlass versehene Verteilerplatte einer Auftragsvorrichtung auf die zur Aufnahme des Wirkstoffs vorgesehene Matrixschicht übertragen wird. Ein solches Verfahren ist aus US-Patent 6 187 322 bekannt.

Der Wirkstoff Nicotin kann mittels der beiden vorstehenden Druckverfahren direkt aufgetragen werden. Erfindungsgemäß wird der Wirkstoff jedoch in Form einer Lösung verwendet, die durch Zugabe des mindestens einen Polymers mit mindestens einer Säureamidgruppe als seitenständiger funktioneller Gruppe die gewünschte Viskosität aufweist. Die Brookfield-Viskosität der als Druckmedium zu verwendenden wirkstoffhaltigen Zusammensetzung liegt vorzugsweise im Bereich von 10 bis 100 dPa·s, besonders bevorzugt in einem Bereich von 15 bis 30 dPa·s, gemessen an einer auf 20 °C temperierten Probe.

Zur Bestimmung der Brookfield-Viskosität wird ein Rotationsviskosimeter, zum Beispiel VT 500 der Firma Haake unter folgenden Bedingungen verwendet: System Nummer 25, Geschwindigkeit 8, Drehkörper ISO 3 d6.

Die Vereinzelung erfolgt bevorzugt so, dass nur außerhalb der Fläche geschnitten und/oder gestanzt wird, auf die die Nicotinlösung aufgedruckt wurde bzw. teilflächig aufgedruckt wurde. Auf diese Weise können produktionsbedingte Wirkstoffverluste weitgehend vermieden werden. Auf diese Weise befindet sich die wirkstoffhaltige Schicht bevorzugt im zentralen Bereich des gebildeten TTS, während der Randbereich des TTS keine wirkstoffhaltige Schicht aufweist.

Das Verfahren zur Herstellung der erfindungsgemäßen TTS ist in einer besonders bevorzugten Ausführungsform dadurch gekennzeichnet, dass man
- ein Laminat aus einer wirkstoffundurchlässigen Trägerschicht (für die ablösbare Schutzschicht), einer haftklebenden Fixierschicht und einer Matrixschicht oder eines Teils der Matrixschicht herstellt,
- mittels eines Druckverfahrens einzeln dosierte Portionen der fließfähigen, wirkstoffhaltigen Zubereitung auf diese Matrixschicht aufbringt, insbesondere teilflächig,
- darauf ggf. eine weitere Matrixschicht oder den restlichen Teil der Matrixschicht laminiert und
- das so erhaltene Laminat schließlich mit einer wirkstoffundurchlässigen Rückschicht versieht,
wobei die transdermalen therapeutischen Systeme durch Schneiden und/oder Stanzen vor oder nach dem Aufbringen der wirkstoffhaltigen Zubereitung aus dem bis dahin entstandenen Verbundlaminat vereinzelt werden können.

Die Vereinzelung erfolgt insbesondere so, dass nur außerhalb der Fläche geschnitten und/oder gestanzt wird, auf die die Nicotinlösung aufgedruckt wurde.

Die Erfindung betrifft ferner die Verwendung eines Polymers mit mindestens einer Säureamidgruppe als seitenständiger funktioneller Gruppe, wobei das Polymer mit mindestens einer Säureamidgruppe als seitenständiger funktioneller Gruppe ein Vinylpyrrolidon-Vinylacetat-Copolymer ist, zur Stabilisierung von Nicotin in einem Nicotin enthaltenden transdermalen therapeutischen System, insbesondere in dem erfindungsgemäßen TTS.

Durch die Stabilisierung des Nicotins wird insbesondere die Verfärbung von Nicotin in dem TTS verlangsamt oder vermieden, die während der Lagerung eines TTS ohne diese Stabilisierung erfolgen würde. Unter Verlangsamung oder Vermeidung der Verfärbung von Nicotin versteht man insbesondere, dass die Verfärbung von farblos nach hellgelb den Farbton Pantone 1215, besonders bevorzugt Pantone 2015 C nicht überschreitet, z.B. nach Aufbewahrung über 3 Monaten unter Lichtausschluss an Luft bei 25°C, bevorzugter bei 40°C und besonders bevorzugt bei 60°C.

Die Erfindung betrifft ferner ein Verfahren zur Stabilisierung von Nicotin in einem Nicotin enthaltenden transdermalen therapeutischen System, umfassend das Beladen des transdermalen therapeutischen Systems mit einem Polymer mit mindestens einer Säureamidgruppe als seitenständiger funktioneller Gruppe, wobei das Polymer mit mindestens einer Säureamidgruppe als seitenständiger funktioneller Gruppe ein Vinylpyrrolidon-Vinylacetat-Copolymer ist, während der Herstellung.

Die Erfindung betrifft weiterhin die Verwendung eines erfindungsgemäßen TTS für ein therapeutisches System auf der Haut, wobei der Wirkstoff in prophylaktisch bzw. therapeutisch wirksamer Menge transdermal abgegeben wird, vorzugsweise über einen Zeitraum von mindestens 24 Stunden.

Das erfindungsgemäße TTS zeigt eine sehr gute Wirkstoffausbeute. So kann der Restnicotingehalt im TTS nach 24-stündiger Benutzung auf der Haut höchstens 60%, betragen (in Umgebung mit Raumtemperatur).

Nachfolgend soll die Erfindung anhand eines Ausführungsbeispiels sowie der begleitenden Zeichnungen, in der schematisch der Aufbau eines Beispiels eines erfindungsgemäßer TTS dargestellt ist, erläutert werden, ohne dass die Erfindung darauf beschränkt wäre. Dabei zeigt:
Fig. 1 einen Schnitt durch eine bevorzugte Ausführungsform eines erfindungsgemäßen TTS; und
Fig. 2 einen Schnitt durch eine weitere bevorzugte Ausführungsform eines therapeutischen Systems, bei dem die wirkstoffhaltige Schicht sich zwischen Rückschicht und Matrixschicht als Reservoir für Nicotin befindet. Dabei ist das TTS jeweils nach Abziehen der ablösbaren Schutzschicht auf die Haut aufgeklebt dargestellt.

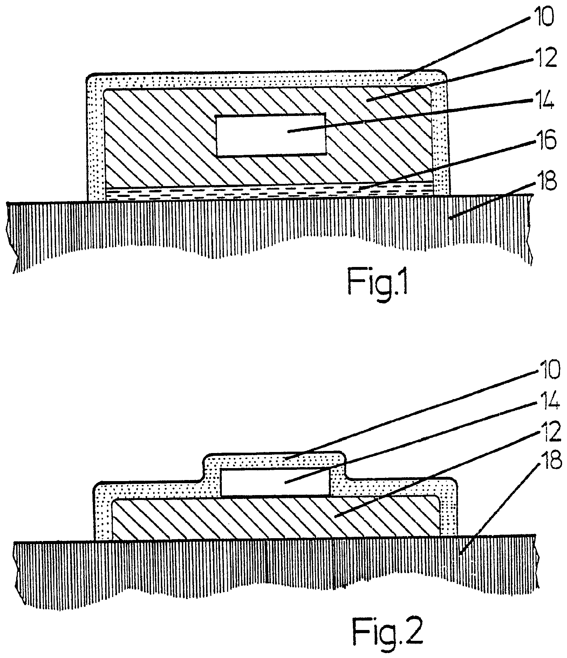

Die wirkstoffhaltige Schicht von Fig. 1 und Fig. 2 umfasst Nicotin und das Polymer mit mindestens einer Säureamidgruppe.

In Fig. 1 ist ein Schnitt durch ein erfindungsgemäßes therapeutisches System, das auf der Haut 18 durch eine haftklebende Fixierungsschicht 16 befestigt ist, schematisch dargestellt. Auf der haftklebenden Schicht 16 befindet sich die Matrixschicht 12, welche bevorzugt zum Zeitpunkt der Herstellung wirkstofffrei ist (die Wirkstoffsättigung tritt während der Lagerung ein). In die Matrixschicht ist eine wirkstoffhaltige Schicht 14 eingebettet, aus der sich Nicotin in die Matrixschicht löst bzw. diffundiert und durch die haftklebende Schicht 16 an die Haut abgegeben wird. Das therapeutische System wird nach außen durch eine transparente Rückschicht 10 abgeschlossen, die für den Wirkstoff Nicotin und bevorzugt auch für Feuchtigkeit undurchlässig ist und gleichzeitig eine Stützfunktion für das System ausübt.

In Fig. 2 befindet sich die wirkstoffhaltige Schicht 14 zwischen der Rückschicht 10 und der Matrixschicht 12. Die Matrixschicht 12 ist haftklebend ausgebildet und auf der Haut 18 befestigt. Alternativ könnte eine haftklebende Schicht (entsprechend der Schicht 16 von Fig. 1) oder eine haftklebende Einrichtung zwischen der Matrixschicht 12 und der Haut 18 angeordnet sein (nicht dargestellt). In diesem Fall ist die Matrixschicht nicht notwendigerweise haftklebend.

### Beispiele

### Referenzbeispiele

Da Polyisobutylene endständige Doppelbindungen enthalten und deshalb durch das Antioxidans Butylhydroxytoluol stabilisiert werden müssen, wurde geprüft, ob die Vermeidung der Nicotinverfärbung durch Antioxidantien erreichen werden kann. Daher wurden in 2 Versuchsreihen pharmazeutisch akzeptable Antioxidantien in abgestuften Konzentrationen zu Lösungen von Eudragit E 100 (kationisches Copolymer auf Basis von Dimethylaminoethylmethacrylat, Butylmethacrylat und Methylmethacrylat in einem Verhältnis von 2:1:1) in Nicotin gegeben. Diese Lösungen wurden unter Lichtausschluss für 4 Wochen bei 60°C bzw. 80 °C gelagert.

Als Antioxidationsmittel wurden Butylhydroxytoluol (BHT), Ascorbylpalmitat und Tocopherol getestet. In Tabelle 1 sind die Zusammensetzungen der getesteten Proben zusammengestellt.

**Tabelle 1: Nicotin Eudragit E 100 Lösungen, die bei 60°C und 80°C gelagert wurden**

| | Referenz | Referenz* | Nic 0001 | Nic 0002 | Nic 0003 | Nic 0004 | Nic 0005 | Nic 0006 | Nic 0007 | Nic 0008 | Nic 0009 | Nic 0010 |
|---|---|---|---|---|---|---|---|---|---|---|---|---|
| Stoff | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. |
| Nicotin | 58,30 | 58,30 | 99,95 | 99,85 | 99,50 | 99,00 | 99,95 | 99,85 | 99,50 | 99,95 | 99,85 | 99,50 |
| Eudraqit E 100 | 41,70 | 41,70 | | | | | | | | | | |
| Butyl hyd roxytoluol(BHT) | / | / | 0,05 | 0,15 | 0,50 | 1,00 | / | / | / | / | / | / |
| Ascorbylpalmitat | / | / | / | / | / | / | 0,05 | 0,15 | 0,50 | / | / | / |
| Tocopherol (VitE) | / | / | / | / | / | / | / | / | / | 0,05 | 0,15 | 0,50 |

| | | | | | | | | | | | | |
|---|---|---|---|---|---|---|---|---|---|---|---|---|
| *N₂ geflutet | | | | | | | | | | | | |

Die Proben wurden nach Lagerung visuell geprüft und der zugehörige Pantone Colour Code für die Farbe der Probe ermittelt wurde. Der Pantone Colour Code ist ein international verbreitetes Farbsystem der Firma Pantone LLC, USA. Als Ergebnis kann festgehalten werden, dass die Antioxidantien die Verfärbung des Nicotins nicht unterdrücken konnten. Beispielsweise zeigten die Nicotin/Eudragit Lösung mit 0,05, 0,15 und 0,5% Ascorbylpalmitat (AP, Nic 0005 - Nic 0007) bei 80°C nach 4 Wochen eine deutliche Braunfärbung:

| | |
|---|---|
| Nic 0005 (+ 0,05% AP) | Pantone Colour Code: 7580C |
| Nic 0006 (+ 0,15% AP) | Pantone Colour Code: 7675C |
| Nic 0007 (+ 0,5% AP) | Pantone Colour Code: 7589C |

Um zu prüfen, welche Zersetzungsprodukte des Nicotins die Braunfärbung verursachen, wurde Probe Nic 0007 analysiert. Die Ergebnisse sind in Tabelle 2 zusammengefasst.

**Tab. 2: Vergleich Abbauprodukte (Mittelwerte aus n=3) [%]**

| **Probe** | **Cotinin** | **Myosmin** | **Unbekannte** |
|---|---|---|---|
| Referenz | 0,06 | 0,13 | < 0,05 |
| Nic0001 | 0,05 | 0,15 | < 0,05 |
| Nic0007 | 0,03 | 0,09 | < 0,05 |

Man erkennt, dass Ascorbylpalmitat nicht die Braunfärbung des Nicotins verhindert, aber seine Zersetzung.

Daher wurde auch Irganox im Vergleich zu BHT auf Nicotin stabilisierende Wirkung untersucht, wobei niedrigere Temperaturen zur Lagerung verwendet wurden. Die Zusammensetzungen und Mischverhältnisse der Proben sind in Tabelle 3 gezeigt.

**Tabelle 3: Nicotin Eudragit E 100 Lösungen , die bei 25°C, 40 °C und 60°C gelagert wurden**

| | Referenz | Referenz Nicotin | Nic + BHT | Nic + Iraan. | 997Nic 0001 | 997Nic 0002 | 997Nic 0003 | 997Nic 0004 | 997Nic 0005 | 997Nic0 006 | 997Nic0 007 | 997Nic0 008 |
|---|---|---|---|---|---|---|---|---|---|---|---|---|
| Stoff | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. | [%] Gew./ Gew. |
| Nicotin | 58,30 | 100,0 | 99,0 | 99,0 | 99,995^{a} | 99,99 ^{a} | 99,97 ^{a} | 99,91 ^{a} | 99,97 ^{a} | 99,91 ^{a} | 99,5 ^{a} | 99,0 ^{a} |
| Eudragit E 100 | 41,70 | / | / | / | | | | | | | | |
| Butyl hyd roxytoluol (BHT) | / | / | 1,0 | / | 0,005 | 0,01 | 0,03 | 0,09 | / | / | / | / |
| Irganox® 1010 | / | / | / | 1,0 | / | / | / | / | 0,03 | 0,09 | 0,5 | 1,0 |

| | | | | | | | | | | | | |
|---|---|---|---|---|---|---|---|---|---|---|---|---|
| ^{a} Verhältnis Nicotin (58,30 Gew./Gew.%) und Eudragit E 100 (41,70 Gew./Gew.%) | | | | | | | | | | | | |

Als Ergebnis kann festgehalten werden, dass auch in dieser Versuchsreihe die Verfärbung des Nicotins nicht unterdrückt werden konnte. Beispielsweise zeigte Nicotin Eudragit E 100 mit 0,03 % Irganox nach 3 monatiger Lagerung folgende Verfärbung:
997Nic0005-1 gelagert bei 25 °C Pantone Colour Code: 120C
997Nic0005-3 gelagert bei 40 °C Pantone Colour Code: 7549C
997Nic0005-5 gelagert bei 60 °C Pantone Colour Code: 7618C

Da offensichtlich die Verfärbung des Nicotins nicht durch Antioxidantien unterdrückt werden kann, wurde nach anderen Wegen gesucht. In Nicotin wurden neutrale Polymere gelöst, um für das Druckverfahren hohe Viskosität zu erreichen.

Es wurden die nachstehend genannten Polymere in Mischung mit Nicotin getestet. Die Mischungen wurden bei 25°C, 40 °C und 60°C über einen Zeitraum von 3 Monaten unter Lichtausschluss an Luft gelagert. Die Zusammensetzungen und Mischverhältnisse der Proben sind in Tabelle 4 gezeigt.

Eingesetzte Polymere für Mischung mit Nicotin:
- Plastoid B: Methylmethacrylat-Butylmethacrylat-Copolymer (1:1) von Evonik
- Eudragit L100-55: Methacrylsäure-Ethylacrylat-Copolymer (1:1) von Evonik Industries
- Siliconöl 12500 cST: Siliconöl
- Povidon K-90: Polyvinylpyrrolidon, Kollidon® 90 F von BASF
- Povidon VA 64: Vinylpyrrolidon-Vinylacetat-Copolymer, Kollidon® VA 6:4 von BASF

**Tabelle 4: Nicotin Polymer Lösungen, die bei 25°C, 40 °C und 60°C gelagert wurden**

| | Plastoid B¹ | Eudragit L100-55**¹ | Siliconöl 12500 cSt**¹ | Povidon K-90¹ | 997Nic 0009¹ | 997Nic 0010**¹ | 997Nic 0011**¹ | 997Nic 0013¹ | 997 Nic0014*¹ | 536 Nic0002 |
|---|---|---|---|---|---|---|---|---|---|---|
| Stoff | [%] Gew./ Gew. | [%] Gew./Gew. | [%] Gew./Gew. | [%] Gew ./Gew. | [%] Gew./Gew. | [%] Gew ./Gew. | [%] Gew./Gew. | [%] Gew ./Gew. | [%] Gew ./Gew. | [%] Gew./Gew. |
| Nicotin | / | / | / | | 58,30 | 58,30 | 58,30 | 90,0 | 100,0 | 67,0 |
| Plastoid B | 100,0 | / | / | / | 41,70 | / | / | / | / | / |
| Eudragit L100-55 | / | 100,0 | / | / | / | 41,70 | / | / | / | / |
| Siliconöl 12500 cST | / | / | 100,0 | / | / | / | 41,70 | / | / | / |
| Povidon K-90 | / | / | / | 100,0 | / | / | / | 10,0 | / | / |
| Povidon VA 64 | / | / | / | / | / | / | / | / | / | 33,0 |

| | | | | | | | | | | |
|---|---|---|---|---|---|---|---|---|---|---|
| * reines Nicotin ** Eudragit L100-55 und Siliconöl 12500 cST sind mit Nicotin nicht kompatibel ¹: Referenzbeispiel | | | | | | | | | | |

Eudragit L100-55 und Siliconöl 12500 cST sind mit Nicotin nicht kompatibel. Plastoid B zeigt zeigt Phasentrennung.

Bei Nicotin mit 10 % PVP (Kollidon K-90 F) ist nach 3 monatiger Lagerung selbst das Muster, das bei 60°C gelagert wurde, nur wenig verfärbt. Die bei 25°C und 40°C gelagerten Proben sind noch schwächer verfärbt und praktisch unverändert. Nachstehend sind die Farben gemäß Pantone Colour Code für die Muster mit PVP angeführt:
997Nic0013-1 initial Pantone Colour Code: 7401C
997Nic0013-1 gelagert bei 25 °C Pantone Colour Code: 2015C
997Nic0013-3 gelagert bei 40 °C Pantone Colour Code: 2015C
997Nic0013-5 gelagert bei 60 °C Pantone Colour Code: 7549C

Die Nicotin Eudragit E 100 Lösung hat unmittelbar nach der Herstellung den Farbton Pantone 1205 und verfärbt sich innerhalb von 3 Monaten bei 25° bzw. 40°C nach Pantone 1215 C, während bei 60 °C braun erreicht wird.

Nicotinlösungen mit PVP VA 64 starten wie die PVP Lösungen bei 7401C und erreichen unabhängig von der Lagertemperatur 25°C, 40°C oder 60 °C nur in den Pantonefarbton 1215 C.

Daher wurden ein Nicotin TTS hergestellt, bei dem zur Viskositätserhöhung der Nicotinbase nicht Eudragit E 100, sondern Vinylpyrrolidon-Vinylacetat-Copolymer (Kollidon® VA 6:4 (Gewichtsverhältnis von Vinylpyrrolidon zu Vinylacetat von 60 zu 40, von BASF)) verwendet wurde.

### Beispiel

Es wird zunächst eine Haftklebermasse HS hergestellt durch Homogenisieren von
a) 933 g eines Handelsproduktes (®Duro-Tak 387-2516 der Fa. Henkel, Düsseldorf, Deutschland - das ist eine 40 %ige Lösung eines selbstvernetzenden Acrylatpolymeren auf Basis von 2-Ethylhexylacrylat, Vinylacetat, Acrylsäure und Titanchelatester in einem Lösungsmittelgemisch aus Essigsäureethylester, Ethanol, Heptan und Methanol) mit
b) 8 g eines Triglycerids fraktionierter Kokosfettsäuren (C8-C10; ®Miglyol 812 der Fa. Evonik Witten, Deutschland).

Daneben werden 6210 g ®Duro-Tak 387-2516, 553 g Essigsäureethylester und 311 g Ethanol mit 66 g des zuvor genannten Triglyerids sowie 626 g eines Acrylharzes aus Dimethylaminoethylmethacrylat und neutralen Methacrylsäureestern (®Eudragit E 100 der Fa. Röhm-Pharma, Darmstadt, Deutschland) versetzt und homogenisiert (Klebermasse MS).

Daneben werden 33 g Vinylpyrrolidon-Vinylacetat-Copolymer (Kollidon® VA 64) in 66 g Nicotin eingetragen und darin gelöst. Es resultiert die Wirkstoffzubereitung.

Die Haftklebermasse HS wird so auf eine abhäsiv ausgerüstete Schutzschicht (A) aufgetragen, dass nach Abdampfen der Lösemittel eine Haftkleberschicht mit einem Flächengewicht von 40 g/m2 gebildet wird.

Die Klebermasse MS wird so auf eine andere abhäsiv ausgerüstete Schutzschicht (B) aufgetragen, dass nach Abdampfen der Lösemittel ein Film mit einem Flächengewicht von 220 g/m2 entsteht. Dieser Film wird auf die auf der Schutzschicht (A) aufgebrachte Haftkleberschicht aufkaschiert. Es resultiert die Unterbahn.

In einem weiteren Beschichtungsgang wird die Klebermasse MS so auf eine weitere abhäsiv ausgerüstete Schutzschicht (C) aufgetragen, dass nach Abdampfen der Lösemittel ein Film mit einem Flächengewicht von 110 g/m2 entsteht, auf den die für den Wirkstoff undurchlässige transparente Rückschicht aufkaschiert wird. Es wird dabei die Oberbahn gebildet.

Nach dem Abziehen der abhäsiv ausgerüsteten Schutzschicht (B) von der Unterbahn wird die Wirkstoffzubereitung mittels eines eiförmigen Silikon-Schaumgummitampons mit einer Shore-Härte von 6 auf die Kleberbahn gedruckt. Die Menge der Wirkstoffzubereitung wird so bemessen, dass jedes TTS später 30 mg nicotinisches Vinylpyrrolidon-Vinylacetat-Copolymer enthält.

Die Oberbahn wird nach Abziehen der abhäsiv ausgerüsteten Schutzschicht (C) auf die Unterbahn (ausgerüstet mit dotierter Wirkstoffzubereitung) aufkaschiert, und es werden TTS ausgestanzt.

Es wird ein TTS gemäß dem schematischen Aufbau von Figur 1 erhalten.

## Patentansprüche

1. Transdermales therapeutisches System (TTS), umfassend
a) eine für Nicotin undurchlässige Rückschicht,
b) eine wirkstoffhaltige Schicht, umfassend Nicotin als Wirkstoff, wobei das Nicotin als freie Base vorliegt, und mindestens ein Polymer mit mindestens einer Säureamidgruppe als seitenständiger funktioneller Gruppe, wobei das Polymer mit mindestens einer Säureamidgruppe als seitenständiger funktioneller Gruppe ein Vinylpyrrolidon-Vinylacetat-Copolymer ist, und
c) eine ablösbare Schutzschicht.

2. Transdermales therapeutisches System nach Anspruch 1, wobei das Vinylpyrrolidon-Vinylacetat-Copolymer teilweise hydrolysiert ist.

3. Transdermales therapeutisches System nach Anspruch 1 oder 2, welches 5 bis 400 mg, vorzugsweise 10 bis 300 mg, insbesondere 14 bis 150 mg Nicotin enthält.

4. Transdermales therapeutisches System nach einem der Ansprüche 1 bis 3, wobei auf der Oberfläche der ablösbaren Schutzschicht, die der Rückschicht zugewandt ist, eine Schicht oder Fixiereinrichtung angeordnet ist, die haftklebend ist, wobei es sich bei der haftklebenden Schicht oder Fixiereinrichtung um die wirkstoffhaltige Schicht oder eine davon verschiedene haftklebende Schicht oder Fixiereinrichtung handeln kann.

5. Transdermales therapeutisches System nach einem der Ansprüche 1 bis 4, wobei das transdermale therapeutische System ferner d) eine Matrixschicht zur Steuerung der Abgabe des Wirkstoffs umfasst, wobei die Matrixschicht eine haftklebende Schicht ist und/oder das transdermale therapeutische System ferner e) eine haftklebende Schicht oder Fixierungseinrichtung umfasst, die zwischen der ablösbaren Schutzschicht und der Matrixschicht angeordnet ist.

6. Transdermales therapeutisches System nach Anspruch 5, umfassend die Matrixschicht und die haftklebende Schicht oder Fixierungseinrichtung zwischen der ablösbaren Schutzschicht und der Matrixschicht.

7. Transdermales therapeutisches System nach einem der Ansprüche 1 bis 6, wobei die wirkstoffhaltige Schicht eine haftklebende Schicht ist.

8. Transdermales therapeutisches System nach einem der Ansprüche 4 bis 7, worin die Schicht oder Fixiereinrichtung, die haftklebend ist, einen Haftklebstoff umfassend mindestens ein Polymer ausgewählt aus natürlichen oder synthetischen Kautschuken, Poly(meth)acrylaten, Polyestern, Polychloroprenen, Polyisobutenen, Polyvinylethern, Polyurethanen, Polyvinylacetaten, Ethylen-Vinylacetat-Copolymeren, Styrol-Dien-Copolymeren wie Styrol-Butadien-Blockcopolymeren, und Silikonen oder einen Heißschmelzkleber umfasst.

9. Transdermales therapeutisches System nach einem der Ansprüche 1 bis 8, worin die für Nicotin undurchlässige Rückschicht transparent ist.

10. Transdermales therapeutisches System nach einem der Ansprüche 1 bis 9, worin der Anteil an Säure in dem transdermalen therapeutischen System ohne Rückschicht und ablösbarer Schutzfolie nicht mehr als 2 Gew.-%, bevorzugt nicht mehr als 0,5 Gew.-%, bevorzugter nicht mehr als 0,02 Gew.-%, bezogen auf das Gewicht des transdermalen therapeutischen Systems ohne Rückschicht und ablösbarer Schutzfolie, beträgt, wobei die wirkstoffhaltige Schicht bevorzugt im wesentlichen frei von Säure ist.

11. Transdermales therapeutisches System nach einem der Ansprüche 1 bis 10, wobei das Gewichtsverhältnis von Nicotin zu dem mindestens einen Polymer mit mindestens einer Säureamidgruppe als seitenständiger funktioneller Gruppe im Bereich von 10 : 1 bis 1 : 2, bevorzugt 5 : 1 bis 1 : 1, liegt.

12. Transdermales therapeutisches System nach einem der Ansprüche 1 bis 11, wobei das transdermale therapeutische System transparent ist.

13. Transdermales therapeutisches System nach einem der Ansprüche 1 bis 12, wobei das Gewichtsverhältnis von Vinylpyrrolidon zu Vinylacetat in dem Vinylpyrrolidon-Vinylacetat-Copolymer 60 zu 40 beträgt.

14. Verfahren zur Herstellung eines transdermalen therapeutischen Systems nach einem der Ansprüche 1 bis 13, wobei das Verfahren folgende Schritte umfasst
- Bereitstellen einer ersten Trägerschicht oder Herstellen eines Ausgangslaminats umfassend eine erste Trägerschicht,
- Aufbringen einer fließfähigen wirkstoffhaltigen Zusammensetzung, umfassend Nicotin als Wirkstoff, wobei das Nicotin als freie Base vorliegt, mindestens ein Polymer mit mindestens einer Säureamidgruppe als seitenständiger funktioneller Gruppe, wobei das Polymer mit mindestens einer Säureamidgruppe als seitenständiger funktioneller Gruppe ein Vinylpyrrolidon-Vinylacetat-Copolymer ist, auf die erste Trägerschicht oder das Ausgangslaminat und
- Auflaminieren der restlichen Schichten des transdermalen therapeutischen Systems, umfassend eine zweite Trägerschicht, auf die mit der wirkstoffhaltigen Zusammensetzung versehene erste Trägerschicht oder das mit der wirkstoffhaltigen Zusammensetzung versehene Ausgangslaminat,
wobei die transdermalen therapeutischen Systeme durch Schneiden und/oder Stanzen vor oder nach dem Aufbringen der wirkstoffhaltigen Zusammensetzung aus dem bis dahin entstandenen Produkt oder Laminat vereinzelt werden können, und die erste Trägerschicht die ablösbare Schutzschicht, die vorzugsweise für Nicotin undurchlässig ist, und die zweite Trägerschicht die für Nicotin undurchlässige Rückschicht bildet oder umgekehrt.

15. Verwendung eines Polymers mit mindestens einer Säureamidgruppe als seitenständiger funktioneller Gruppe, wobei das Polymer mit mindestens einer Säureamidgruppe als seitenständiger funktioneller Gruppe ein Vinylpyrrolidon-Vinylacetat-Copolymer ist, zur Stabilisierung von Nicotin in einem Nicotin enthaltenden transdermalen therapeutischen System, bevorzugt, um die Verfärbung von Nicotin zu verlangsamen oder zu vermeiden.

16. Verfahren zur Stabilisierung von Nicotin in einem Nicotin enthaltenden transdermalen therapeutischen System, umfassend das Beladen des transdermalen therapeutischen Systems mit einem Polymer mit mindestens einer Säureamidgruppe, als seitenständige funktionelle Gruppe, wobei das Polymer mit mindestens einer Säureamidgruppe als seitenständige funktionelle Gruppe ein ein Vinylpyrrolidon-Vinylacetat-Copolymer ist, während der Herstellung.

## Claims

1. A transdermal therapeutic system (TTS), comprising
a) a back layer, impermeable to nicotine,
b) an active agent-containing layer, comprising nicotine as active agent, wherein the nicotine is in the form of a free base, and at least one polymer having at least one acid amide group as pendant functional group, wherein the polymer having at least one acid amide group as pendant functional group is a vinylpyrrolidone-vinyl acetate copolymer, and
c) a releasable protective layer.

2. The transdermal therapeutic system according to claim 1, wherein the vinylpyrrolidone-vinyl acetate copolymer is partially hydrolysed.

3. The transdermal therapeutic system according to claim 1 or 2, which contains 5 to 400 mg, preferably 10 to 300 mg, in particular 14 to 150 mg of nicotine.

4. The transdermal therapeutic system according to one of claims 1 to 3, wherein a layer or fixing means, which is self-adhesive, is arranged on the surface of the releasable protective layer, which faces the back layer, wherein the self-adhesive layer or fixing means can be the active agent-containing layer or a self-adhesive layer or fixing means different of it.

5. The transdermal therapeutic system according to one of claims 1 to 4, wherein the transdermal therapeutic system further comprises d) a matrix layer for controlling the release of the active agent, wherein the matrix layer is a self-adhesive layer and/or the transdermal therapeutic system further comprises e) a self-adhesive layer or fixing means which is arranged between the releasable protective layer and the matrix layer.

6. The transdermal therapeutic system according to claim 5, comprising the matrix layer and the self-adhesive layer or fixing means between the releasable protective layer and the matrix layer.

7. The transdermal therapeutic system according to one of claims 1 to 6, wherein the active agent-containing layer is a self-adhesive layer.

8. The transdermal therapeutic system according to one of claims 4 to 7, wherein the layer or fixing means, which is self-adhesive, comprises a pressuresensitive adhesive comprising at least one polymer selected from natural or synthetic rubbers, poly(meth)acrylates, polyesters, polychloroprenes, polyisobutenes, polyvinyl ethers, polyurethanes, polyvinyl acetates, ethylenevinyl acetate copolymers, styrene-diene copolymers, such as styrene-butadiene block copolymers, and silicones, or a hot-melt adhesive.

9. The transdermal therapeutic system according to one of claims 1 to 8, wherein the back layer impermeable to nicotine is transparent.

10. The transdermal therapeutic system according to one of claims 1 to 9, wherein the proportion of acid in the transdermal therapeutic system without back layer and releasable protective film is no more than 2% by weight, preferably no more than 0.5% by weight, more preferably no more than 0.02% by weight, based on the weight of the transdermal therapeutic system without back layer and releasable protective film, wherein the active agent-containing layer is preferably substantially free from acid.

11. The transdermal therapeutic system according to one of claims 1 to 10,
wherein the weight ratio of nicotine to the at least one polymer having at least one acid amide group as pendant functional group is in the range of from 10:1 to 1:2, preferably 5:1 to 1:1.

12. The transdermal therapeutic system according to one of claims 1 to 11,
wherein the transdermal therapeutic system is transparent.

13. The transdermal therapeutic system according to one of claims 1 to 12,
wherein the weight ratio of vinylpyrrolidone to vinyl acetate in the vinylpyrrolidone-vinyl acetate copolymer is 60 to 40.

14. A method for producing a transdermal therapeutic system according to one of claims 1 to 13, wherein the method comprises the following steps
- providing a first support layer or producing a starting laminate comprising a first support layer,
- applying a flowable active agent-containing composition, comprising nicotine as active agent, wherein the nicotine is in the form of a free base, at least one polymer having at least one acid amide group as pendant functional group, wherein the polymer having at least one acid amide group as pendant functional group is a vinylpyrrolidone-vinyl acetate copolymer, onto the first support layer or the starting laminate, and
- laminating the remaining layers of the transdermal therapeutic system, comprising a second support layer, onto the first support layer provided with the active agent-containing composition or the starting laminate provided with the active agent-containing composition,
wherein the transdermal therapeutic systems can be isolated by being cut and/or punched from the product or laminate produced up to that point, before or after the application of the active agent-containing composition, and the first support layer forms the releasable protective layer, which preferably is impermeable to nicotine, and the second support layer forms the back layer impermeable to nicotine, or vice versa.

15. Use of a polymer having at least one acid amide group as pendant functional group, wherein the polymer having at least one acid amide group as pendant functional group is a vinylpyrrolidone-vinyl acetate copolymer, for stabilising nicotine in a nicotine-containing transdermal therapeutic system, preferably in order to slow down or avoid the discolouration of nicotine.

16. A method for stabilising nicotine in a nicotine-containing transdermal therapeutic system, comprising the charging of the transdermal therapeutic system with a polymer having at least one acid amide group as pendant functional group, wherein the polymer having at least one acid amide group as pendant functional group is a vinylpyrrolidone-vinyl acetate copolymer, during the production.

## Revendications

1. Système thérapeutique transdermique (TTS) comprenant
a) une couche arrière imperméable à la nicotine,
b) une couche contenant un principe actif, comprenant de la nicotine en tant que principe actif, dans lequel la nicotine est présente en tant que base libre, et au moins un polymère avec au moins un groupe amide d'acide en tant que groupe fonctionnel autonome, dans lequel le polymère avec au moins un groupe amide d'acide en tant que groupe fonctionnel autonome est un copolymère de vinylpyrrolidone-acétate de vinyle, et
c) une couche de protection détachable.

2. Système thérapeutique transdermique selon la revendication 1, dans lequel le copolymère de vinylpyrrolidone-acétate de vinyle est en partie hydrolysé.

3. Système thérapeutique transdermique selon la revendication 1 ou 2, lequel contient 5 à 400 mg, de préférence 10 à 300 mg, en particulier 14 à 150 mg de nicotine.

4. Système thérapeutique transdermique selon l'une quelconque des revendications 1 à 3, dans lequel est disposée ou disposé sur la surface de la couche de protection détachable, qui est tournée vers la couche arrière, une couche ou un dispositif de fixation, qui est adhésive ou adhésif, dans lequel la couche adhésive ou le dispositif de fixation adhésif peut être la couche contenant un principe actif ou une couche adhésive ou un dispositif de fixation adhésif différente ou différent de celle-ci.

5. Système thérapeutique transdermique selon l'une quelconque des revendications 1 à 4, dans lequel le système thérapeutique transdermique comprend en outre d) une couche de matrice pour commander la distribution du principe actif, dans lequel la couche de matrice est une couche adhésive et/ou le système thérapeutique transdermique comprend en outre e) une couche adhésive ou un dispositif de fixation adhésif, qui est disposée ou disposé entre la couche de protection détachable et la couche de matrice.

6. Système thérapeutique transdermique selon la revendication 5, comprenant la couche de matrice et la couche adhésive ou le dispositif de fixation adhésif entre la couche de protection détachable et la couche de matrice.

7. Système thérapeutique transdermique selon l'une quelconque des revendications 1 à 6, dans lequel la couche contenant un principe actif est une couche adhésive.

8. Système thérapeutique transdermique selon l'une quelconque des revendications 4 à 7, dans lequel la couche ou le dispositif de fixation qui est adhésive ou adhésif comprend une matière adhésive comprenant au moins un polymère choisi parmi des caoutchoucs naturels ou synthétiques, des poly(méth)acrylates, des polyesters, des polychloroprènes, des polyisobutènes, des éthers polyvinyliques, des polyuréthanes, des acétates de polyvinyle, des copolymères d'éthylène-acétate de vinyle, des copolymères de styrène-diène tels que copolymères en bloc de styrène-butadiène, et des silicones ou une colle thermofusible.

9. Système thérapeutique transdermique selon l'une quelconque des revendications 1 à 8, dans lequel la couche arrière imperméable à la nicotine est transparente.

10. Système thérapeutique transdermique selon l'une quelconque des revendications 1 à 9, dans lequel la fraction en acide dans le système thérapeutique transdermique sans couche arrière et sans film de protection détachable ne dépasse pas 2 % en poids, de manière préférée 0,5 %, de manière plus préférée 0,02 % en poids, par rapport au poids du système thérapeutique transdermique sans couche arrière et sans film de protection détachable, dans lequel la couche contenant un principe actif est de manière préférée sensiblement sans acide.

11. Système thérapeutique transdermique selon l'une quelconque des revendications 1 à 10, dans lequel le rapport de poids entre la nicotine et l'au moins un polymère avec au moins un groupe amide d'acide en tant que groupe fonctionnel autonome se situe dans la plage de 10:1 à 1:2, de manière préférée de 5:1 à 1:1.

12. Système thérapeutique transdermique selon l'une quelconque des revendications 1 à 11, dans lequel le système thérapeutique transdermique est transparent.

13. Système thérapeutique transdermique selon l'une quelconque des revendications 1 à 12, dans lequel le rapport de poids entre la vinylpyrrolidone et l'acétate de vinyle dans le copolymère de vinylpyrrolidone-acétate de vinyle est de 60 à 40.

14. Procédé de fabrication d'un système thérapeutique transdermique selon l'une quelconque des revendications 1 à 13, dans lequel le procédé comprend des étapes suivantes :
- de fourniture d'une première couche de support ou de fabrication d'un stratifié de départ comprenant une première couche de support,
- d'application d'une composition pouvant s'écouler contenant un principe actif, comprenant de la nicotine en tant que principe actif, dans lequel la nicotine est présente en tant que base libre, au moins un polymère avec au moins un groupe amide d'acide en tant que groupe fonctionnel autonome, dans lequel le polymère avec au moins un groupe amide d'acide en tant que groupe fonctionnel autonome est un copolymère de vinylpyrrolidone-acétate de vinyle, sur la première couche de support ou le stratifié de départ, et
- d'application par laminage des couches restantes du système thérapeutique transdermique, comprenant une deuxième couche de support, sur la première couche de support pourvue de la composition contenant un principe actif ou le stratifié de départ pourvu de la composition contenant un principe actif,
dans lequel les systèmes thérapeutiques transdermiques peuvent être isolés par découpage et/ou estampage avant ou après l'application de la composition contenant un principe actif du produit ou stratifié s'y étant formé, et la première couche de support forme la couche de protection détachable, qui de préférence est imperméable à la nicotine, et la deuxième couche de support forme la couche arrière imperméable à la nicotine, ou inversement.

15. Utilisation d'un polymère avec au moins un groupe amide d'acide en tant que groupe fonctionnel autonome, dans laquelle le polymère avec au moins un groupe amide d'acide en tant que groupe fonctionnel autonome est un copolymère de vinylpyrrolidone-acétate de vinyle, pour stabiliser la nicotine dans un système thérapeutique transdermique contenant de la nicotine, de manière préférée pour ralentir ou éviter la coloration de la nicotine.

16. Procédé de stabilisation de la nicotine dans un système thérapeutique transdermique contenant de la nicotine, comprenant le chargement du système thérapeutique transdermique avec un polymère avec au moins un groupe amide d'acide en tant que groupe fonctionnel autonome, dans lequel le polymère avec au moins un groupe d'amide d'acide en tant que groupe fonctionnel autonome est un copolymère de vinylpyrrolidone-acétate de vinyle, au cours de la fabrication.
